(19) **Europäisches Patentamt / European Patent Office / Office européen des brevets**

(11) **EP 4 481 641 A1**

(12) **EUROPEAN PATENT APPLICATION**
published in accordance with Art. 153(4) EPC

(43) Date of publication:
**25.12.2024  Bulletin 2024/52**

(21) Application number: **23759499.9**

(22) Date of filing: **12.01.2023**

(51) International Patent Classification (IPC):
***G06N 99/00*** (2019.01)

(52) Cooperative Patent Classification (CPC):
**G06N 99/00**

(86) International application number:
**PCT/JP2023/000530**

(87) International publication number:
**WO 2023/162493 (31.08.2023 Gazette 2023/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **28.02.2022  JP 2022030366**

(71) Applicants:
- **Calmarion Inc.**
  **Tokyo 150-0002 (JP)**
- **Inui, Koji**
  **Tokyo 113-0033 (JP)**

(72) Inventor: **INUI, Koji**
**Tokyo 113-0033 (JP)**

(74) Representative: **Patentanwaltskanzlei WILHELM
& BECK**
**Prinzenstraße 13**
**80639 München (DE)**

(54) **INFORMATION PROCESSING DEVICE, INFORMATION PROCESSING METHOD, AND PROGRAM**

(57)    In order to facilitate obtainment of a desired property quantity by optimization of a system, an information processing device (1) includes: a property quantity calculating section (11) that calculates, from a Hamiltonian ($H(\theta)$) that is defined by a manipulable parameter ($\theta$), a property quantity ($P(\theta)$) of a system ($S$) that is defined by the Hamiltonian; and an updating section (12) that updates the parameter so as to increase a value of an objective function ($L(P)$) that is related to the property quantity and that includes the parameter as an argument or so as to decrease the value of the objective function.

FIG. 1

EP 4 481 641 A1

**Description**

Technical Field

The present invention relates to an information processing device, an information processing method, and a program that are for optimizing a system.

Background Art

There has been known a technique for optimizing a substance system. For example, Patent Literature 1 discloses a technique for designing a substance on the basis of quantum-mechanical calculation.

Citation List

[Patent Literature]

[Patent Literature 1]
Japanese Patent Application Publication Tokukai No. 2016-69302

Summary of Invention

Technical Problem

However, it is not easy to obtain desired properties by optimizing a system. In general, in order to optimize a substance system, it is necessary to repeat (i) identifying elements of a substance system and (ii) find properties of the substance system on the basis of quantum-mechanical calculation.
Objects of an aspect of the present invention is to provide an information processing device, an information processing method, and a program that are to (a) efficiently search for a substance and/or a model which expresses a desired property quantity by optimizing a system and (b) implement, by an algorithm, discovery of a substance and/or a model which no person could arrive at intuitively.

Solution to Problem

In order to solve the above problem, an information processing device in accordance with an aspect of the present invention includes: a property quantity calculating section that calculates, from a Hamiltonian that is defined by a manipulable parameter, a property quantity of a system that is defined by the Hamiltonian; and an updating section that updates the parameter so as to increase a value of an objective function that is related to the property quantity and that includes the parameter as an argument or so as to decrease the value of the objective function.

Advantageous Effects of Invention

An aspect of the present invention makes it possible to provide an information processing device, an information processing method, and a program that are to (a) efficiently search for a substance and/or a model which expresses a desired property quantity by optimizing a system and (b) implement, by an algorithm, discovery of a substance and/or a model which no person could arrive at intuitively.

Brief Description of Drawings

Fig. 1 is a block diagram illustrating a configuration of an information processing device in accordance with an embodiment of the present invention.
Fig. 2 is a diagram illustrating an example of a system.
Fig. 3 is a flowchart showing a flow of an optimization process that is carried out by an information processing device in accordance with an embodiment of the present invention.
Fig. 4 is a chart showing a relation between a parameter and a property quantity.
Fig. 5 is a flowchart showing a flow of an optimization process that is carried out by an information processing device in accordance with a variation of the present invention.

Fig. 6 is a block diagram illustrating a hardware configuration example of an information processing device in accordance with an embodiment of the present invention.

Description of Embodiments

[Embodiments]

The following description will discuss in detail an embodiment of the present invention. An information processing device in accordance with the present embodiment is to optimize a system so that a desired property quantity can be obtained.

(Information processing device)

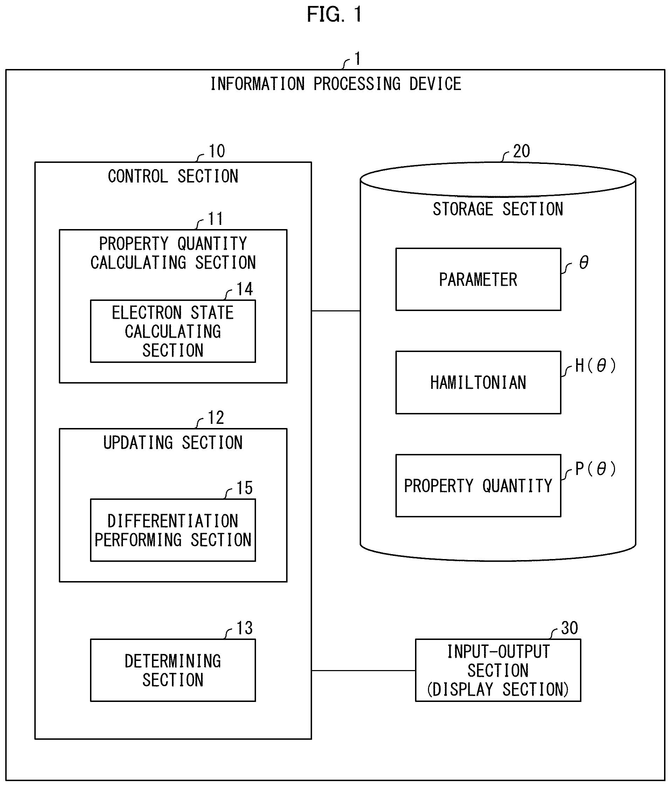

The following will specifically describe a configuration of an information processing device with reference to Fig. 1. Fig. 1 is a block diagram illustrating a configuration of the information processing device 1 in accordance with the present embodiment. The information processing device 1 includes a control section 10, a storage section 20, and an input-output section 30, as illustrated in Fig. 1.

(Storage section)

First, the following description will discuss various data stored in the storage section 20, with reference to Fig. 1. As illustrated in Fig. 1, in the storage section 20, a parameter θ, a Hamiltonian H(θ), and a property quantity P(θ) are stored.
The parameter θ is a manipulable parameter in a given system S. The system S includes various systems, for example, any physical system or chemical system such as a substance system or an optical system. The system S is basically composed of an atom (including a molecule) or a combination of atoms/molecules.

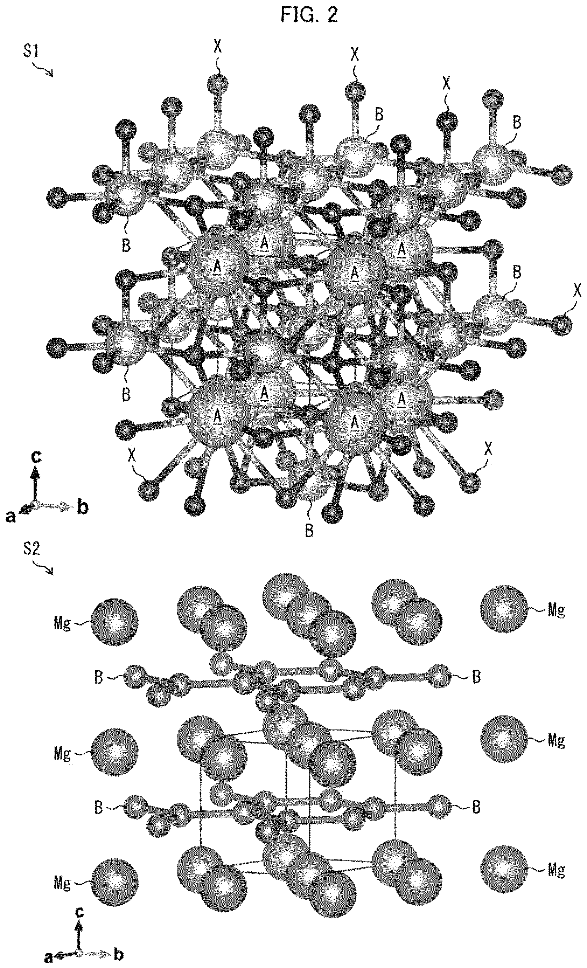

Fig. 2 is a diagram illustrating an example of the system S. Here, the system S refers to a system S1 and a system S2. The system S1 shows a unit lattice of $ABX_3$ in which sites A, B, and C are combined. Respective atoms (molecules) disposed in sites A, B, and C differ from each other. In the site A, for example, $CH_3NH_3^+$, $Cs^+$, or $HC(NH_2)_2^+$ is disposed. In the site B, for example, $Pb^{2+}$ or $Sn^{2+}$ is disposed. In the site X, for example, I-, Br-, or Cl- is disposed. In this way, a type and an arrangement of atoms (in the present case, ions) can be represented by a lattice. In the system S2, a steric arrangement of atoms Mg and B is shown.
The system S is defined by the parameter θ. The parameter θ is a variable element that influences properties of the system S and that is defined by a numerical value. Examples of the parameter θ include a temperature of the system S, or a magnetic field, electric field, or pressure applied to the system S. Further, the type, combination, and arrangement of atoms, an atomic distance, and a valence (the number of electrons) expressed in numerical value each can be also used as the parameter θ. In other words, the parameter θ corresponds to at least one selected from, for example, the group consisting of: the type, combination, and arrangement of atoms constituting the system S; the atomic distance; the number of electrons; the temperature of the system S; and the magnetic field, the electric field, and the pressure applied to the system S.
The Hamiltonian H(θ) is an operator corresponding to energy of the system S, and is inextricably linked with the system S. The Hamiltonian H(θ) is used to calculate an electron state of the system S by an electron state calculating section which will be described later. The Hamiltonian H is expressed by, for example, the following expression (1).

$$\hat{H}\Psi = \left[\hat{T} + \hat{V} + \hat{U}\right]\Psi = \left[\sum_{i=1}^{N}\left(-\frac{\hbar^2}{2m_i}\nabla_i^2\right) + \sum_{i=1}^{N}V(\mathbf{r}_i) + \sum_{i<j}^{N}U\left(\mathbf{r}_i,\mathbf{r}_j\right)\right]\Psi = E\Psi,$$

The expression (1) is an equation (Schrödinger equation) of a wave function Ψ including the Hamiltonian H that is a first principal. The Hamiltonian H is expressed as the sum of a kinetic energy T, a potential energy V, and an interaction energy U (H = T + V + U). Note that ri and rj correspond to the position of an electron.
It is possible to express the Hamiltonian H(θ) as a function of the parameter θ, by (i) adding, to the Hamiltonian H(θ) of the expression (1), a term that varies in accordance with the parameter θ or (ii) taking into consideration an influence of the parameter θ on at least one selected from the group consisting of the kinetic energy T, the potential energy V, and the interaction energy U. For example, the system S has an energy state that changes as result of a change in the temperature of the system S, or a magnetic field, an electric field, an electromagnetic wave, an acoustic wave or a pressure applied to the system S. Further, the type, combination, and arrangement of atoms constituting the system S, the atomic distance, and the valence can cause a change in the energy state of the system S, and can be incorporated in the Hamiltonian H(θ) as the parameter θ. In this way, the parameter θ is a variable that can take consecutive numerical numbers that are selected from elements of the system S (i.e., the Hamiltonian H).

Here, it is possible to use a model Hamiltonian H which is simplified as in expression (2), in place of the Hamiltonian H expressed as expression (1) which is a first principal.

$$\mathcal{H} = \sum_{k\mu\nu} t_{k\mu\nu} c^{\dagger}_{k\mu} c_{k\nu} + \sum_{\substack{k_1 k_2 k_3 k_4 \\ \mu\nu\xi\lambda}} V_{k_1 k_2 k_3 k_4} c^{\dagger}_{k_1\mu} c_{k_2\nu} c^{\dagger}_{k_3\xi} c_{k_4\lambda}$$

$$+ \sum_q \omega_q a^{\dagger}_q a_q + \sum_{qk\mu} g_q (c^{\dagger}_{k+q\mu} c_{k\mu} a_q + c^{\dagger}_{k\mu} c_{k+q\mu} a^{\dagger}_q)$$

In the model Hamiltonian, it is possible to use, as the parameter $\theta$ of the Hamiltonian H($\theta$), a term of hopping between electrons, an electron-electron interaction term, an electron-lattice interaction term, an effective coupling constant (coefficient of a term of three or higher order in the Hamiltonian), an effective overlap integral (term that occurs due to an overlap of electron orbits), and an effective interaction (modeled interaction such as an interaction between spins which is derived from the Hamiltonian that is a first principal).

In the storage section 20, various functions that define such a Hamiltonian H($\theta$) and a plurality of parameters $\theta$ of the functions are stored.

The property quantity P($\theta$) is a quantity that represents properties (feature) of the system S, and varies in accordance of the parameter $\theta$. In other words, the property quantity P($\theta$) is a function of the parameter $\theta$. Examples of the property quantity P($\theta$) include linear and non-linear responses (e.g., Hall coefficient) with respect to a superconducting transition temperature, a superconducting gap, thermoelectric efficiency, photovoltaic power generation efficiency, and external fields (e.g., magnetic field, electric field, electromagnetic wave, acoustic wave, and pressure). The storage section 20 stores therein various equations for deriving such a property quantity P($\theta$), on the basis of the electron state of the system S which is derived from the Hamiltonian.

(Input-output section)

The input-output section 30 receives data from outside the information processing device 1 or outputs data to the outside of the information processing device 1. For example, the input-output section 30 includes an input device such as a keyboard or a touchpad, and receives input of data via such an input device. Then, the received data is supplied to the control section 10. Further, for example, the input-output section 30 includes a display panel, and displays, through the display panel, data supplied from the control section 10. In this case, the input-output section 30 functions as the display section, and displays at least one selected from the group consisting of a value of the parameter $\theta$, a value of the property quantity P, and a value of an objective function L which will be described later, for example, a relation between the parameter $\theta$ and the property quantity P($\theta$).

(Control section)

Next, the following will discuss a configuration of the control section 10 included in the information processing device 1, with reference to Fig. 1. The control section 10 includes a property quantity calculating section 11, an updating section 12, and a determining section 13, as illustrated in Fig. 1. The property quantity calculating section 11 includes an electron state calculating section 14, and the updating section 12 includes a differentiation performing section 15.

The property quantity calculating section 11 calculates, as below, the property quantity P($\theta$) of the system S that is defined by the Hamiltonian H($\theta$), from the Hamiltonian H($\theta$) that is defined by a manipulable parameter $\theta$.

First, the electron state calculating section 14 of the property quantity calculating section 11 calculates an electron state of the system S on the basis of the Hamiltonian H($\theta$). In the case of the Hamiltonian H expressed by the expression (1), which is a first principal, the electron state calculating section 14 calculates the electron state by, for example, the Hartree-Fock approximation method or the density function method. In the case of the model Hamiltonian which is expressed by the expression (2), the electron state calculating section 14 calculates the electron state of the system S by general numerical calculation.

Next, the property quantity calculating section 11 calculates the property quantity P on the basis of the electron state which has been calculated. For example, the property quantity P can be calculated by determining a band structure, a lattice configuration, a phonon dispersion, or a force field on the basis of the electron state. A linear response or a non-linear response may be used for calculating the property quantity P. For example, it is possible to use, as the property quantity P, for example, a Hall coefficient which is calculated from the band structure with use of a linear response theory, or a nonlinear optical conductivity which is calculated from the band structure with use of a nonlinear response theory. Further, it is possible to use, as the property quantity P, a superconducting gap that is calculated from, for example, the band structure and the phonon dispersion.

The updating section 12 updates the parameter $\theta$ so as to increase a value of an objective function $L(P(\theta))$ which is relevant to the property quantity $P(\theta)$ and which includes the parameter $P(\theta)$ as an argument or so as to decrease the value of the objective function.

The objective function $L(P)$ can be set as follows in accordance with a target of the property quantity $P(\theta)$.

In a case where it is desired to increase or decrease the property quantity P, the objective function $L(P)$ is set to be a function proportional to the property quantity P ($L = A \cdot P$, A: positive or negative proportionality constant). In a case where it is desired to increase the property quantity P, the proportionality constant A is set to be positive (e.g., $L(P) = P$, where A = 1). On the other hand, in a case where it is desired to decrease the property quantity P, the proportionality constant A is set to be negative (e.g., $L(P) = -P$, where A = -1).

In a case where it is desired to bring the property quantity P closer to a target value Pt, for example, the objective function $L(P)$ is set to be a function of a difference between the property quantity P and the target value Pt of the property quantity P ($L = function(P - Pt)$). Specifically, the objective function $L(P)$ is set to "$L(P) = (P - Pt)^2$".

In some cases, the property quantity P is a spectral property quantity $P(x)$ representing dependency of properties on a physical quantity x, and it is desired to bring the spectral property quantity $P(x)$ closer to a target spectral property quantity $Pt(x)$. In this case, for example, the objective function $L(P)$ is set to be a function ($L(P) = \int(P(x) - Pt(x))dx$) obtained by integrating, in terms of the physical quantity x, a difference between the spectral property quantity $P(x)$ and the target spectral property quantity $Pt(x)$ which is a target of the spectral property quantity.

The differentiation performing section 15 performs differentiation ($\partial P/\partial \theta$) which relates to the parameter $\theta$ and which is differentiation of the property quantity $P(\theta)$. In other words, the differentiation performing section 15 calculates a value (differential value) of the differentiation ($\partial P/\partial \theta$). This differential value ($\partial P/\partial \theta$) is used for updating the parameter $\theta$. Specifically, the differentiation performing section 15 performs automatic differentiation ($\partial P/\partial \theta$) as follows.

That is, in each calculation process that is decomposed to a level at which analytic differentiation can be performed, a differential value thereof is retained in a memory. With use of the differential value, $\partial P/\partial \theta$ is calculated by an automatic differentiation technique.

Note that a specific algorithm for performing the automatic differentiation is not intended to limit the present embodiment. For example, the differentiation performing section 15 can be configured to perform the automatic differentiation by executing a differentiation algorithm that is used in the error back-propagation method (Back Propagation) of a neural network.

The updating section 12 updates the parameter $\theta$ so that the value of the objective function $L(P(\theta))$ becomes larger or smaller. In a case where the parameter $\theta$ is updated from a value $\theta_i$ to a value $\theta_{i+1}$, it is possible to derive the value $\theta_{i+1}$ from the value $\theta_i$ as shown in expression (3), for example, with use of the steepest descent method. The expression (3) indicates that the parameter $\theta$ can be updated with use of the value of the differentiation ($\partial P/\partial \theta$) that has been calculated by the differentiation performing section 15.

$$\theta_{i+1}$$
$$= \theta_i - \alpha \cdot [\partial L(P(\theta))/\partial\theta] \cdot \theta_i$$
$$= \theta_i - \alpha \cdot [\partial L(P)/\partial P] \cdot [\partial P(\theta)/\partial\theta] \cdot \theta_i \quad \dots \quad (3)$$

$\alpha$: proportionality constant

More preferably, the updating section 12 uses an optimization technique such as stochastic gradient descent (SGD), Adam, RMSPROP, K-Fac, or EK-Fac. In any case, the updating section 12 can update the parameter $\theta$ with use of the value of the differentiation $\partial L/\partial\theta$ which has been calculated by the differentiation performing section 15.

The determining section 13 determines whether to end updating of the parameter $\theta$ by comparing parameters $\theta$ (value $\theta_{i+1}$ and $\theta_i$) before and after updating. This determination means determination of convergence of the parameter $\theta$. For example, it is possible to determine whether to end updating of the parameter $\theta$ on the basis of the following expression (4).

$$|\theta_{i+1} - \theta_i| =< Th \quad \dots \quad (4)$$

In other words, the determining section 13 determines to end updating of the parameter $\theta$ in a case where the absolute value of the difference between the values $\theta_{i+1}$ and $\theta_i$ of the parameter $\theta$ before and after updating is equal to or less than a threshold value Th. On the other hand, the determining section 13 determines not to end updating of the parameter $\theta$ in a case where the absolute value of the difference is larger than the threshold value Th.

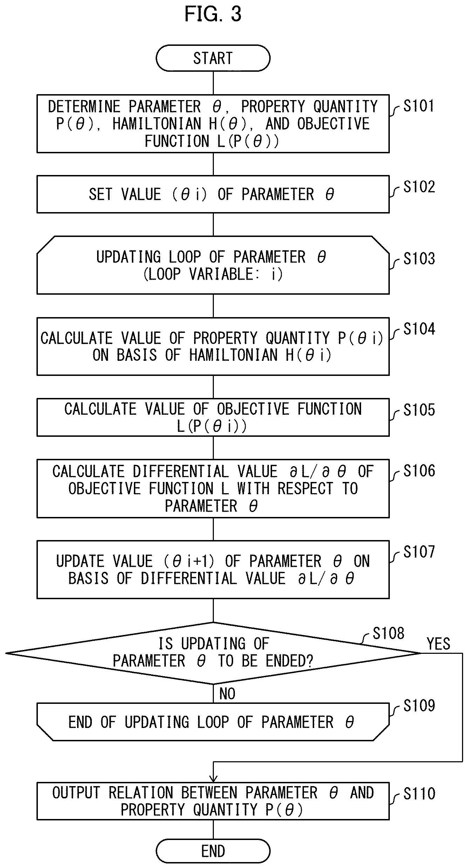

The following description will discuss an operation of the information processing device 1. Fig. 3 is a flowchart showing a flow of an optimization process that is carried out by an information processing device in accordance with an embodiment of the present invention.

(1) Determination of parameter $\theta$, property quantity $P(\theta)$, Hamiltonian $H(\theta)$, and objective function $L(P(\theta))$ (step S101)

Determining the parameter $\theta$, the property quantity $P(\theta)$, and the Hamiltonian $H(\theta)$ means selecting a combination of the parameter $\theta$, the Hamiltonian $H(\theta)$, and the property quantity $P(\theta)$ that are stored in the storage section 20. As a result, the Hamiltonian $H(\theta)$ (ultimately, the system S) is selected as an object in which a desired property P is searched for.

The objective function $L(P(\theta))$ can be selected as a function of the property quantity $P(\theta)$ as appropriate, on the basis of a target for the property quantity $P(\theta)$, as described above.

(2) Setting of value $\theta_i$ of parameter $\theta$ (step S102) The value $\theta_i$ is set for the parameter $\theta$. A default value can be set for the value $\theta_i$ as appropriate.

(3) Calculation of value $P_i$ of property quantity $P(\theta_i)$ on basis of Hamiltonian $H(\theta_i)$ (steps S103 and S104)

The electron state calculating section 14 calculates an electron state of the system S on the basis of the Hamiltonian $H(\theta)$. The property quantity calculating section 11 calculates the value $P_i$ of the property quantity P on the basis of the electron state which has been calculated. Step S104 corresponds to a property quantity calculation step in which the property quantity $P(\theta)$ of the system S that is defined by the Hamiltonian $H(\theta)$ is calculated from the Hamiltonian $H(\theta)$ that is defined by a manipulable parameter $\theta$.

(4) Calculation of value of objective function $L(P(\theta_i))$ (step S105)

A value $L_i$ of the function $L(P)$ is calculated by substituting the value $P_i$ of the property quantity P into the function $L(P)$.

(5) Calculation of differential value $\partial L(P(\theta))/\partial \theta$ of objective function $L(P)$ of property quantity P with respect to parameter $\theta$ (step S106)

The differentiation performing section 15 calculates a differential value $\partial P/\partial \theta$ of the property quantity $P(\theta)$ which is related to the parameter $\theta$. The updating section 12 calculates a differential value $\partial L(P(\theta))/\partial \theta$ on the basis of the differential value $\partial P/\partial \theta$ (see expression (3): $\partial L(P(\theta))/\partial \theta = [\partial L(P)/\partial P]\cdot[\partial P(\theta)/\partial \theta]$). Step S106 corresponds to an update step of updating the parameter $\theta$ so that the property quantity P calculated in the property quantity calculation step approaches a desired value.

(6) Calculation of value $\theta_{i+1}$ of the parameter $\theta$ for maximizing or minimizing function $L(P(\theta))$ (step S108)

The updating section 12 updates the parameter $\theta$ from the value $\theta_i$ to the value $\theta_{i+1}$, for example, on the basis of the expression (3).

(7) Determination of whether to end updating of parameter $\theta$ (step S109)

The determining section 13 determines whether or not it is possible to end updating of the parameter $\theta$. In other words, whether the function $L(P(\theta))$ is in a convergence state is evaluated. Specifically, in a case where the difference between the value $\theta_{i+1}$ and the value $\theta_i$ is equal to or less than the threshold value Th, the function $L(P(\theta))$ is determined to be in the convergence state. On the other hand, in a case where the difference is larger than the threshold value Th, the function $L(P(\theta))$ is determined to be in a non-convergence state.

In the case of the convergence state, the optimization process is ended. The value $\theta_i$ of the parameter $\theta$ at this time is considered to be suitable for setting the property quantity P of the system S to a desired state.

The information processing device 1 can display, via the input-output section 30, the value $\theta_i$ of the parameter $\theta$, the property quantity $P(\theta_i)$, or the objective function $L(P(\theta_i))$ each obtained as described above or a relation of these.

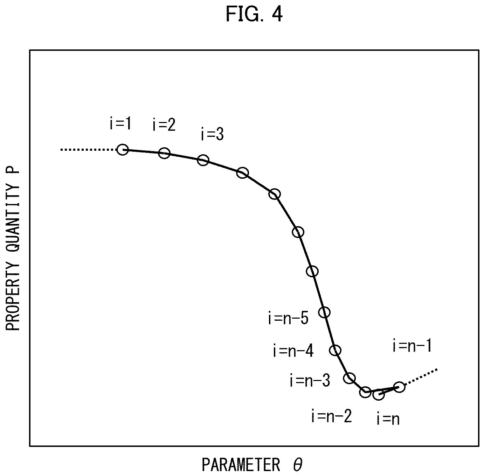

Fig. 4 is a chart showing a relation between the parameter and the property quantity. The parameter $\theta$ and the

property quantity P change in accordance with a loop variable i. Here, in a range in which the loop variable i is relatively small, the parameter θ increases and the property quantity P decreases as the loop variable i increases. When the loop variable i changes from n - 2 to n - 1, both of the parameter θ and the property quantity P increase. Thereafter, when the loop variable i changes from n-1 to n, both of the parameter θ and the property quantity P decrease and the parameter θ is determined to have been converged (in this case, the property quantity P is substantially a minimum value). In this way, in accordance with an increase in the loop variable i, the parameter θ repeatedly varies so as to approach the target of the property quantity P.

As described above, according to the present embodiment, a desired property quantity P(θ) can be obtained by optimizing the Hamiltonian H(θ) (system S) by updating the parameter θ.

[Variation]

The following description will discuss a variation of the present invention. In the above-described embodiment, for easy understanding, use of only one parameter θ was assumed. However, it is possible to use two or more parameters θ. In other words, it is possible to optimize the system S with respect to the property quantity P, by changing a plurality of parameters θ. In this case, the plurality of parameters θ can be changed in parallel, but the plurality of parameters θ can alternatively be sequentially changed.

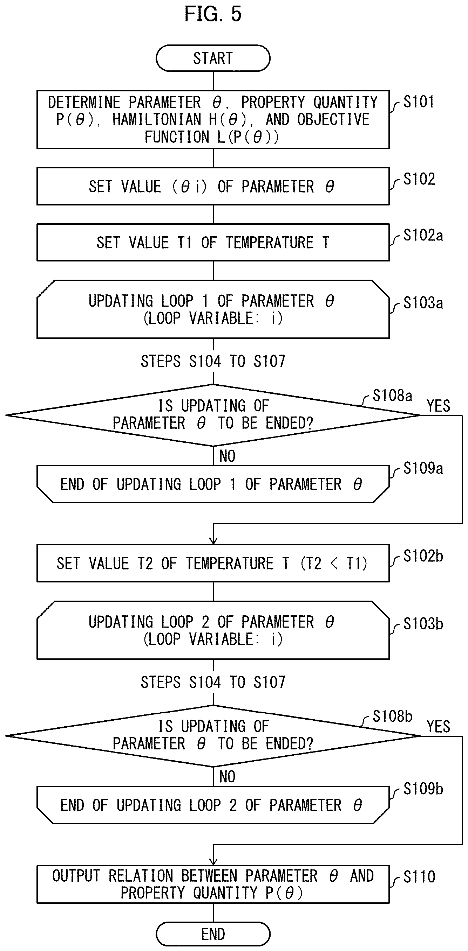

Fig. 5 is a flowchart showing a flow of an optimization process that is carried out by an information processing device in accordance with a variation of the present invention. In this flow, a temperature T is used as the parameter θ.

Here, after setting the value $θ_i$ of the parameter θ (step S102), a value T1 of the temperature T of the system S is set (step S102a). Then, an updating loop 1 of another parameter θ is executed (steps S103a, S104 to S107, S108a, and S109a). In the updating loop 1, when updating of the parameter θ is determined to be ended (step S108a), a value T2 of the temperature T of the system S is set (step S102b). Then, an updating loop 2 of the parameter θ is executed (steps S103b, S104 to S107, S108b, and S109b).

Here, it is preferable that a condition (e.g., threshold Th1) for determining the end in step S108a be milder than another condition (e.g., threshold Th2) for determining the end in step S108b (e.g., Th1 > Th2). This is because: the updating loop 1 serves as a preceding stage of the updating loop 2; and the updating loop 1 is for narrowing down a range of search in the updating loop 2.

The temperature T1 set in the step S102a is higher than the temperature T2 set in the step S102b. In other words, the parameter θ is converged by decreasing the temperature from an initial temperature. Such a technique is effective in a case where calculation of the property quantity P includes a linear response or a non-linear response. In such a case, a differentiation quantity $\partial P/\partial θ$ may not be stable, and the parameter θ may be difficult to converge. By increasing the temperature T1 that is an initial temperature, a stable differentiation quantity $\partial P/\partial θ$ can be obtained, and thus updating of the parameter θ and consequent convergence of the parameter θ can be facilitated.

Here, for easy understanding, the temperature is changed in two stages, but the temperature may be changed in three or more stages. For example, the temperature is first set to a high temperature and then it is possible to advance the optimization while gradually decreasing the temperature.

Any of various elements can be used as the parameter θ, provided that such an element is a variable element that influences properties of the system S and that is defined by a numerical value. Examples of the parameter θ include (1) to (3) below. Note that in the case of (3), the electron state calculating section 14 can calculate the electron state with use of, for example, virtual crystal approximation (VCA).

(1) A frequency-dependent magnetic field, electric field, electromagnetic wave, acoustic wave, and the like
(2) A composition ratio of atoms (e.g., x of a molecule $(A_xB_{1-x})CO_3$)
(3) A combination of two or more kinds of atoms

The parameter θ may be a configuration of a lattice. Further, the parameter θ may be a force field that influences the configuration of the lattice. In this case, it is possible to optimize a structure of the lattice, by updating the configuration of the lattice by setting the force field as the parameter θ.

(Hardware configuration example of information processing device 1)

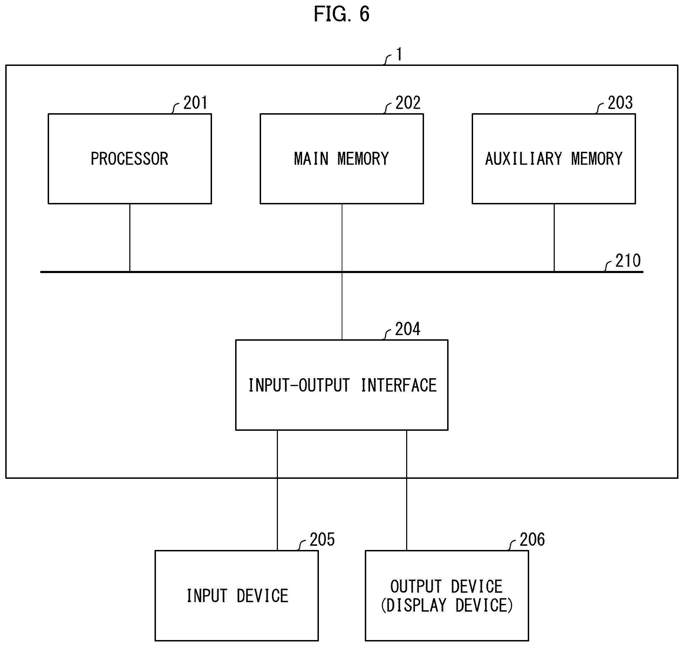

Fig. 6 is a block diagram illustrating a hardware configuration example of a computer that is used as the information processing device 1. The information processing device 1 can be configured by a computer that includes a bus 210, a processor 201, a main memory 202, an auxiliary memory 203, a communication interface 204, and an input-output interface 205, as illustrated in Fig. 6. The processor 201, the main memory 202, the auxiliary memory 203, the communication interface 204, and the input-output interface 205 are connected to each other via the bus 210. With respect to the input-output interface 205, the input device 206 and the output device 207 are connected.

Examples of the processor 201 encompass a central processing unit (CPU), a microprocessor, a digital signal processor, a microcontroller, a graphic processing unit (CPU), a tensor processing unit (TPU), or a combination thereof.

As the main memory 202, for example, a semiconductor random access memory (RAM) is used.

As the auxiliary memory 203 may be, for example, a flash memory, a hard disk drive (HDD), a solid state drive (SSD), or a combination thereof. A program for causing the processor 201 to carry out an operation of the information processing device 1 described above is stored in the auxiliary memory 203. The processor 201 causes the program stored in the auxiliary memory 203 to be loaded in the main memory 202 and executes instructions contained in the loaded program.

The communication interface 204 connects to a network N.

The input-output interface 205 can be, for example, a universal serial bus (USB) interface, a near field communication interface such as an infrared interface and Bluetooth (registered trademark), or any combination of these interfaces.

The input device 206 can be, for example, a keyboard, a mouse, a touchpad, a microphone, or any combination of these devices. The output device 207 can be, for example, a display, a printer, a speaker, or any combination of these devices.

In this example, the processor 201 is an example of a hardware element that realizes the control section 10. Further, the main memory 202 and the auxiliary memory 03 are each an example of the hardware element that realizes the storage section 20. Furthermore, the input-output interface 205, the input device 206, and the output device 207 are each an example of the hardware element that realizes the above-described input-output section 30.

[Software Implementation Example]

Functions of the information processing device 1 can be realized by a program for causing a computer to function as the device, the program causing the computer to function as control blocks (in particular, sections included in the control section 10) of the device.

In this case, the device includes, as hardware for executing the program, a computer that includes at least one control device (e.g., a processor) and at least one storage device (e.g., a memory). By executing the program with the control device and the storage device, the functions described in the above embodiments are realized.

The program can be stored in at least one computer-readable non-transitory storage medium. The storage medium can be provided in the device, or the storage medium does not need to be provided in the device. In the latter case, the program can be supplied to the device via an arbitrary wired or wireless transmission medium.

Further, one or some or all of respective functions of the control blocks described above can be realized by a logic circuit. For example, an integrated circuit in which a logic circuit that functions as each of the control blocks is formed is also encompassed in the scope of the present invention.

In addition, one or some or all of respective functions of the control blocks described above can be operated by the control device or another device (e.g., an edge computer or a cloud server).

Aspects of the present invention can also be expressed as follows:

The whole or part of the example embodiments disclosed above can also be described as below. Note, however, that the present invention is not limited to the following example aspects.

An information processing device (1) in accordance with Aspect 1 of the present invention includes: a property quantity calculating section (11) that calculates, from a Hamiltonian ($H(\theta)$) that is defined by a manipulable parameter ($\theta$), a property quantity ($P(\theta)$) of a system (S) that is defined by the Hamiltonian; and an updating section (12) that updates the parameter so as to increase a value of an objective function ($L(P)$) that is related to the property quantity and that includes the parameter as an argument or so as to decrease the value of the objective function. With the above configuration, it is possible to optimize the property quantity of a system by updating the parameter.

An information processing device in accordance with Aspect 2 of the present invention can be configured such that in the above Aspect 1, the updating section includes a differentiation performing section (15) that performs automatic differentiation ($\partial P/\partial\theta$) which is differentiation of the property quantity and which is related to the parameter. With the above configuration, it is possible to calculate the value ($\theta_{i+1}$) of the parameter after updating by using the differential value ($\partial P/\partial\theta_i$) obtained with use of the value of the parameter ($\theta_i$) before the updating.

An information processing device in accordance with Aspect 3 of the present invention may be configured to further include, in the above Aspect 1 or 2, a determining section (13) that determines whether to end updating of the parameter by comparing parameters before and after the update. With the above configuration, it is possible to determine convergence of the parameter and end updating of the parameter.

An information processing device in accordance with Aspect 4 of the present invention can be configured such that in any one of Aspects 1 to 3, the property quantity calculating section includes an electron state calculating section (14) that calculates an electron state of the system, on the basis of the Hamiltonian. With the above configuration, the property quantity can be calculated on the basis of the electron state which has been calculated.

An information processing device in accordance with Aspect 5 of the present invention can be configured such that in any one of Aspects 1 to 4, the parameter corresponds to: a type, a combination, or an arrangement of atoms constituting the system; an atomic distance; the number of electrons; an effective coupling constant; an effective overlap integral; an effective interaction; a temperature of the system; or a magnetic field, an electric field, an electromagnetic wave, an acoustic wave, or a pressure applied to the system. With the above configuration, it is possible to use, as a parameter, atoms constituting the system, a pressure applied to the system, or the like.

An information processing device in accordance with Aspect 6 of the present invention can be configured such that in any one of Aspects 1 to 5, the property quantity is a linear or non-linear response to a superconducting transition temperature, a superconducting gap function, thermoelectric efficiency, photovoltaic power generation efficiency, or an external field. With the above configuration, the superconducting transition temperature or the like can be calculated as the property quantity.

An information processing device in accordance with Aspect 7 of the present invention can be configured such that in any one of Aspects 1 to 6, the objective function is proportional to the property quantity. With the above configuration, it is possible to increase or decrease the property quantity by updating the parameter.

An information processing device in accordance with Aspect 8 of the present invention can be configured such that in any one of Aspects 1 to 6, the objective function is a function of a difference between the property quantity and a target value of the property quantity. With the above configuration, it is possible to bring the property quantity closer to the target value.

An information processing device in accordance with Aspect 9 of the present invention can be configured such that in any one of Aspects 1 to 6, the property quantity is a spectral property quantity representing dependency of a property on a physical quantity; and the objective function is a function obtained by integrating, in terms of the physical quantity, a difference between the spectral property quantity and a target spectral property quantity which is a target of the spectral property quantity. With the above configuration, it is possible to bring the spectral property quantity closer to the target spectral property quantity by updating the parameter.

An information processing device in accordance with Aspect 10 of the present invention can be configured to further include, in any one of Aspects 1 to 9, a display section (input-output section 30) that displays at least one selected from the group consisting of a value of the parameter, a value of the property quantity, and a value of the objective function. With the above configuration, it is possible to present a transition of any one of the parameter, the property quantity, and the objective function obtained as a result of updating.

An information processing method in accordance with Aspect 11 of the present invention includes: a property quantity calculation step of calculating, from a Hamiltonian that is defined by a manipulable parameter, a property quantity of a system that is defined by the Hamiltonian; and an update step of updating the parameter so that the property quantity calculated in the property quantity calculation step approaches a desired value. With the above configuration, it is possible to optimize the property quantity of a system by updating the parameter.

A program in accordance with Aspect 12 of the present invention is a program for causing a computer to function as the information processing device according to Aspect 1, the program causing the computer to function as the property quantity calculating section and the updating section. With the above configuration, it is possible to optimize the property quantity of a system by updating the parameter.

The present invention is not limited to the embodiments, but can be altered by a skilled person in the art within the scope of the claims. The present invention also encompasses, in its technical scope, any embodiment derived by combining technical means disclosed in differing embodiments.

Reference Signs List

1 information processing device
10 control section
11 property quantity calculating section
12 updating section
13 determining section
14 electron state calculating section
15 differentiation performing section
20 storage section
30 input-output section

**Claims**

1. An information processing device comprising:

   a property quantity calculating section that calculates, from a Hamiltonian that is defined by a manipulable parameter, a property quantity of a system that is defined by the Hamiltonian; and
   an updating section that updates the parameter so as to increase a value of an objective function that is related to the property quantity and that includes the parameter as an argument or so as to decrease the value of the objective function.

2. The information processing device according to claim 1, wherein
   the updating section includes a differentiation performing section that performs automatic differentiation which is differentiation of the property quantity and which is related to the parameter.

3. The information processing device according to claim 1 or 2, further comprising
   a determining section that determines whether to end updating of the parameter by comparing parameters before and after the update.

4. The information processing device according to any one of claims 1 to 3, wherein
   the property quantity calculating section includes an electron state calculating section that calculates an electron state of the system, on the basis of the Hamiltonian.

5. The information processing device according to any one of claims 1 to 4, wherein
   the parameter corresponds to: a type, a combination, or an arrangement of atoms constituting the system; an atomic distance; the number of electrons; an effective coupling constant; an effective overlap integral; an effective interaction; a temperature of the system; or a magnetic field, an electric field, an electromagnetic wave, an acoustic wave, or a pressure applied to the system.

6. The information processing device according to any one of claims 1 to 5, wherein
   the property quantity is a linear or non-linear response to a superconducting transition temperature, a superconducting gap, thermoelectric efficiency, photovoltaic power generation efficiency, or an external field.

7. The information processing device according to any one of claims 1 to 6, wherein
   the objective function is proportional to the property quantity.

8. The information processing device according to any one of claims 1 to 6, wherein
   the objective function is a function of a difference between the property quantity and a target value of the property quantity.

9. The information processing device according to any one of claims 1 to 6, wherein:

   the property quantity is a spectral property quantity representing dependency of a property on a physical quantity; and
   the objective function is a function obtained by integrating, in terms of the physical quantity, a difference between the spectral property quantity and a target spectral property quantity which is a target of the spectral property quantity.

10. The information processing device according to any one of claims 1 to 9, further comprising
    a display section that displays at least one selected from the group consisting of a value of the parameter, a value of the property quantity, and a value of the objective function.

11. An information processing method comprising:

    a property quantity calculation step of calculating, from a Hamiltonian that is defined by a manipulable parameter, a property quantity of a system that is defined by the Hamiltonian; and
    an update step of updating the parameter so that the property quantity calculated in the property quantity calculation step approaches a desired value.

12. A program for causing a computer to function as the information processing device according to claim 1, the program causing the computer to function as the property quantity calculating section and the updating section.

FIG. 1

# FIG. 2

## FIG. 3

START

DETERMINE PARAMETER $\theta$, PROPERTY QUANTITY P($\theta$), HAMILTONIAN H($\theta$), AND OBJECTIVE FUNCTION L(P($\theta$)) — S101

SET VALUE ($\theta$i) OF PARAMETER $\theta$ — S102

UPDATING LOOP OF PARAMETER $\theta$ (LOOP VARIABLE: i) — S103

CALCULATE VALUE OF PROPERTY QUANTITY P($\theta$i) ON BASIS OF HAMILTONIAN H($\theta$i) — S104

CALCULATE VALUE OF OBJECTIVE FUNCTION L(P($\theta$i)) — S105

CALCULATE DIFFERENTIAL VALUE $\partial L/\partial \theta$ OF OBJECTIVE FUNCTION L WITH RESPECT TO PARAMETER $\theta$ — S106

UPDATE VALUE ($\theta$i+1) OF PARAMETER $\theta$ ON BASIS OF DIFFERENTIAL VALUE $\partial L/\partial \theta$ — S107

IS UPDATING OF PARAMETER $\theta$ TO BE ENDED? — S108 — YES

NO

END OF UPDATING LOOP OF PARAMETER $\theta$ — S109

OUTPUT RELATION BETWEEN PARAMETER $\theta$ AND PROPERTY QUANTITY P($\theta$) — S110

END

FIG. 4

# FIG. 5

START

DETERMINE PARAMETER $\theta$, PROPERTY QUANTITY P($\theta$), HAMILTONIAN H($\theta$), AND OBJECTIVE FUNCTION L(P($\theta$)) — S101

SET VALUE ($\theta$i) OF PARAMETER $\theta$ — S102

SET VALUE T1 OF TEMPERATURE T — S102a

UPDATING LOOP 1 OF PARAMETER $\theta$ (LOOP VARIABLE: i) — S103a

STEPS S104 TO S107

IS UPDATING OF PARAMETER $\theta$ TO BE ENDED? — S108a → YES

NO

END OF UPDATING LOOP 1 OF PARAMETER $\theta$ — S109a

SET VALUE T2 OF TEMPERATURE T (T2 < T1) — S102b

UPDATING LOOP 2 OF PARAMETER $\theta$ (LOOP VARIABLE: i) — S103b

STEPS S104 TO S107

IS UPDATING OF PARAMETER $\theta$ TO BE ENDED? — S108b → YES

NO

END OF UPDATING LOOP 2 OF PARAMETER $\theta$ — S109b

OUTPUT RELATION BETWEEN PARAMETER $\theta$ AND PROPERTY QUANTITY P($\theta$) — S110

END

FIG. 6

# EP 4 481 641 A1

## INTERNATIONAL SEARCH REPORT

| | International application No. |
|---|---|
| | **PCT/JP2023/000530** |

### A. CLASSIFICATION OF SUBJECT MATTER

*G06N 99/00*(2019.01)i
FI: G06N99/00 180

According to International Patent Classification (IPC) or to both national classification and IPC

### B. FIELDS SEARCHED

Minimum documentation searched (classification system followed by classification symbols)

G06N99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-201566 A (QUNASYS INC.) 17 December 2020 (2020-12-17) | 1, 7-8, 10-12 |
| | paragraphs [0174]-[0205] | |
| Y | | 2-6 |
| A | | 9 |
| Y | ZHANG, Yu et al., Variational Quantum Eigensolver with Reduced Circuit Complexity, 14 June 2021, pp. 1-12, [retrieved on 10 March 2023], Internet: <URL: https://arxiv.org/pdf/2106.07619v1.pdf> | 2-6 |
| | pp. 3-5 | |
| Y | JP 2012-15427 A (NATIONAL INSTITUTE OF ADVANCED INDUSTRIAL SCIENCE AND TECHNOLOGY) 19 January 2012 (2012-01-19) | 6 |
| | paragraphs [0028]-[0031] | |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 March 2023** | **20 March 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/000530**

| Patent document cited in search report | | | Publication date (day/month/year) | Patent family member(s) | Publication date (day/month/year) |
|---|---|---|---|---|---|
| JP | 2020-201566 | A | 17 December 2020 | (Family: none) | |
| JP | 2012-15427 | A | 19 January 2012 | (Family: none) | |

Form PCT/ISA/210 (patent family annex) (January 2015)

**EP 4 481 641 A1**

**Patent documents cited in the description**

- JP 2016069302 A